# EUROPEAN PATENT SPECIFICATION

(11) **EP 0 678 295 B1**
(45) Date of publication and mention of the grant of the patent: **25.07.2001**
(21) Application number: 95105736.3
(22) Date of filing: 18.04.1995
(51) Int. Cl.: A61K 9/127, B01J 13/00

(54) **Manufacturing process of phospholipideactive principle complexes useful for the liposome preparation**
Herstellungsverfahren für Komplexe aus Phospholipid und Wirkstoff nützlich zur Liposomenherstellung
Procédé de préparation de complexes d'un phospholipide avec une substance active utilisables pour la fabrication de liposomes

(30) Priority: 22.04.1994 IT MI940778
(43) Date of publication of application: 25.10.1995
(73) Proprietor: Citernesi, Ugo, I-20043 Arcore (Milano) (IT)
(72) Inventor: Citernesi, Ugo, I-20043 Arcore (Milano) (IT)
(74) Representative: Cioni, Carlo

(56) References cited:
- EP-A- 0 317 120
- WO-A-90/04961
- WO-A-91/16882
- WO-A-93/23015
- GB-A- 2 013 609
- GB-A- 2 047 535
- US-A- 4 271 196
- US-A- 4 311 712
- US-A- 4 731 210
- US-A- 4 830 858
- US-A- 4 963 362

## Description

The present invention concerns a process for the manufacture of phospholipid - active principle complexes useful in the subsequent preparations of liposomes exhibiting a high active principle encapsulating efficiency.

The liposome formation mechanism from said complexes, which may be in gel, liquid or powder form, is spontaneous and it takes place simply by complex hydration followed by mechanical stirring.

Liposomes exhibit a structure similar to the one of the cell membranes. It is well known that liposomes are bi-layer phospholipid spherical aggregates that are formed in aqueous solutions as a consequence of a mechanical reorganization of the bimolecular layers in which phospholipids spontaneously organize when dispersed in an aqueous solution. The liposome vesicles may consist of a single membrane phospholipid double layer (SUV-Small Unilamellar Vesicles) or two or more membrane phospholipid double layers (MLV- Multilayer Lipid Vesicles) having their polar heads oriented toward the exterior and toward the vesicle inner aqueous layers.

Liposomes have been used in the past in the pharmaceutical field, nowadays they find an extensive use in the cosmetic industry. The interest of said industry in the use of liposome is due to the fact that liposomes may constitute highly effective systems for the delivery of a broad spectrum of hydro- and lipo-soluble active principles. The hydrosoluble materials may be contained into the liposome inner aqueous spaces and electrostatically bound to the lipid double layer surface. On the other hand the liposoluble materials are retained within the lipid double layer or are sometime entrapped, as minute droplets, within the liposome aqueous zones. The use of liposomes as pharmaceutical and cosmetic active principle carriers has some advantages i.e. a higher treatment efficiency, a reduction of the active principle to be administered, an active principle protection against its deterioration as well as a prolonged release time thereof.

At present many liposome manufacturing methods are known in the art. However most of said methods show, among other drawbacks, the difficulty, and more likely the impossibility, to load the active principles into the liposomes. Therefore the researches in this field aimed to find an effective method to manufacture active principles containing liposomes exhibiting a high encapsulating efficiency.

In order to better illustrate the novelty of the present invention, four kind of techniques are reported hereinafter which are presently employed to manufacture loaded liposomes, i.e. one or more active principle containing liposomes.

The first method, known as passive entrapping, implies the liposome vesicles formation as a consequence of the mechanical stirring of an active principle containing phospholipid aqueous dispersion. The active principle is entrapped into the liposome simultaneously with the liposome formation. Said method has the drawback of a reduced encapsulating efficiency (a large amount of the active principle remaining in the aqueous medium and is not entrapped into the liposome) as well as a variable encapsulating activity depending on the dispersion medium nature, the active principle and other substance concentration.

The second method implies the "solvent evaporation" from a two phase solvent system. Said technique, besides of being unable to provide high encapsulating efficiency liposomes, has the further drawback of promoting the presence into the liposomic solution of undesired organic solvent residues, as for instance, chlorinated hydrocarbons, making them unsuitable for pharmaceutical or cosmetic purposes.

The third technique, making use of a gel consisting of membrane phospholipid double layers, is based on a "two phase dilution". In the first phase an active principle concentrated solution in an insufficient amount for the liposome formation, is mixed with the initial gel. In the second phase the resulting solution is then further diluted with an excess aqueous solution without any active principle. This procedure, even successful in obtaining an active principle encapsulating level higher than the previous techniques, still has, however some disadvantages. First the encapsulating efficiency is no higher than 80% and moreover said efficiency is not constant, but it varies depending on the nature of the active principle. Therefore, to obtain active principle prefixed content liposomes - which is a critical requirement for the use in the pharmaceutical field - a long and expensive set of experimental tests is necessary.

A further disadvantage resides in that the dilution should respect precise values to avoid any active principle release from the liposome inner part to the surrounding dispersing aqueous medium.

The recently disclosed fourth technique, is the so-called "transmembrane loading technique". Said technique, disclosed in PCT/EP 91/02377, involves the preliminary preparation of "empty liposomes", i.e. containing the dispersion aqueous medium only. Thereafter the active principle is introduced into the dispersion medium and, by incubation under controlled temperature and osmolality values, the penetration of the active principle into the core of the liposome will occur. Even if with the above technique it is possible to obtain satisfactory values of the encapsulating degree, said degree is still variable in function of the nature of the active principle to be encapsulated into the liposomes, the following documents refer to the liposomes preparation:
WO-A-90 04961 (pag. 14, lines 10-11) discloses a process step in which the active principle/phospholipid mixture is transformed into liposome by addition of saline solution.
GB-A-2 013 609 discloses liposome formation by addition of a saline solution to the lipid film (Example 4) or lipid dispersion (Example 6) The obtained liposome are then forzen and lyophilised.
US-A-4 830 858 disclises the preparation of liposome precursors by spraying-drying the a mixture of liposomal components. There is not mention of any complex formation.
GB-A-2 047 535 discloses the preparation of stable injectable solutions comprising a mixture of indolacetic and indanacetic acid derivatives and phospholipid. There is not any mention of liposome preparation.
USA-A-4 311 712 and US-A-4 963 362 are similar to US-A-4 830 858. The spraying-drying technique is replaced by a freeze-drying process.
WO-A-93 23015 discloses a process in which a lipid mixture is hydrated by an acueous solution of an aminoglycoside to form a liposomal composition, then the liposomal composition is lyophilised for subsequent rehdrating steps.
EP-A-317 120 prepares a complex by acidifying a phosphatidylglycerol to solubilize an amphilic drug. There is a chemical modification of the lipid component.
US-A-4 731 210 discloses a process for liposome preparation in which associates of an amphilic substance and a solubilising agent are formed. Solubilising agent are separated from the associates when liposomes are formed.
WO-A-91 16882 prepares a mixture of a lipid and a solution of a drug and then submit the mixture to spray drying. There is not any mention of a complex formation.
US-A-4 271 196 is directed to the preparation of a lipid vehicle useful to solubilise unsoluble medicaments.
GB-A-2 047 535 discloses a process to prepare a stable injectable solution of phospholipid and a derivative of indolacetic and indanacetic derivative prepared by lyophilising a mixture of said component ( Example 2)

### Summary of the invention.

It is an object of the present invention to provide the preliminary preparation of phospholipid and active principle complexes and then "loaded liposomes" - i.e. active principle containing liposome - by a simple and quick method which does not involve the limitations of the prior art techniques.

The invention, as hereinafter described, consists of a process for the preparation of a phospholipid /active principle complex useful for the manufacture of one or more active substances containing liposome, wherein the active principle, intrinsically bound to the phospholipid molecules, is entrapped within liposomes, comprising the following steps:
a) dissolving the active principle(s) into a solvent,
b) phospholipid addition to the above solution and solvent removing if necessary,
c) final treatment of the complex to obtain the desired form.
characterized in that an alcoholic solution is added to the mixture coming out of step b) to enable the above complex molecules to reorganise in a multilamellar structure having a plurality of bimolecular layers.

The rationale upon which this system is based consists of the preliminary formation of a complex between the active principle and the phospholipids and then the subsequent arrangement in more double layers of the obtained complex. The invention takes advantage of the phospholipid molecule amphipathy, i.e. the fact that said molecules exhibit a hydrophilic portion and a hydrophobic portion, to create complexes both with hydrophilic and hydrophobic active principles, the first bound to the polar head the second to the hydrophobic tail of the phospholipid .

The phospholipid lactive principle complex may be in form of a gel, a liquid or a powder and, by a simple water addition followed by a short mechanical stirring, it allows to obtain liposomes whose active principle, being intrinsically bound to phospholipid molecules, is not merely contained into the liposomic cavity or dissolved in the aqueous phase, but is directly linked to the liposome membrane with an encapsulation degree close to 100%.

### Description of the preferred embodiments.

The present invention provides several advantages over the prior art techniques which are illustrated hereinafter.
1. Possibility of preparing phospholipid /active principle complexes having a component stable relationship.
2. High Liposome active principle encapsulating capacity, close to 100% irrespective of the active principle nature. Therefore it is possible to prepare liposome dispersion having a predetermined content both as active principle and phospholipids. Then, the amount of active principle not encapsulated into the liposome and consequently not useful, being unable to cross the epidermal barrier, is, in practice, reduced to zero.
3. Preservative absence in the phospholipid /active principle complex that avoids any risk of preservative substance liposimizing or encapsulating. In case a preservative containing liposomic solution is required, preservatives shall be added after liposome formation so that they are present in the aqueous dispersion only, but not within liposomes.
4. The possibility of manufacturing apyrogenic, pharmaceutical grade, sterile liposome solution without any preservative addition.
5. Liposome stability, in that no migration of the active principle will occur due to osmotic equilibria involving mechanisms.
6. Possibility of on-the-spot liposome preparation, when required for the use, avoiding therefore any problem connected with liposome conservation and stability. This characteristic enables the preparation of compositions consisting of two components to be mixed when used, that means very stable compositions even in the absence of preservatives useful for the active principle injection or administration to cell cultures.

Convenient active principles useful in the process according to the present invention may vary depending on the sought applications. In the cosmetic field applications the following can be used: anti-wrinkle active principles such as polyunsaturated fatty acids, Echinacea extract, Aloe Vera extract, Fetuin, Vitamin A and the like; free radical scavenging active principles such as Superoxide Dismutase, Peroxidase, Leucocianidins, Vitamin E; cellulitis preventing active principles such as Escin, Ruscogenin, Caffein, Horse-chestnut extract, Camitin and the like, revitalizing active principles as Cytochrome C, placenta extract, Ginseng extract and the like. In the pharmaceutical field applications the following can be used: antimycotic active principles as thiazole derivatives, Griseofulvin and the like; antibiotic active principles as Penicillin, Vancomycin, Cephalosporin and the like; hormone based active principles as Calcitonin. In the dietetic field applications the following can be used: vitamin and other adjuvant based active principles. In the agronomy field applications the following can be used: herbicide active principles as dinitro-ortho-cresol, carbamates and the like; fungicide active principles as organophosphates, organochlorides, thiophosphates and the like.

Convenient phospholipids for the use in the complex manufacturing process according to the invention can be either of animal and vegetal origin or synthetic. Among the vegetal origin phospholipids soybean lecithin can be employed, among the animal origin ones egg lecithin and cerebrosides can be used.

### A - GEL FORM

PHASE 1 The active principle is dissolved in a suitable solvent;

PHASE 2 Preparation of the complex between the active principle solution resulting from the previous phase and the phospholipids, whose nature and purity level is in accordance with to the ultimate use of the product. If the selected solvent (f.i. hexane) should not be present in the final product it will be removed by vacuum distillation or evaporation.

PHASE 3: A multilamellar phospholipid /active principle structure is obtained by addition of an ethanol based solution until a soft mixture is formed.

PHASE 4: gel form completion by glycerol addition.

### B - LIQUID FORM

PHASE 1 AND 2: As indicated under A (gel form)

PHASE 3: A multilamellar phospholipid /active principle structure is obtained by addition of an ethanol based solution in such an amount to yield a liquid product.

### C - POWDER

PHASE 1,2 AND 3: As indicated under A above (gel form).

PHASE 4: The complex is dried under vacuum at a predefined temperature depending on the nature of the active principle.

As aforesaid, to obtain liposomes, it is sufficient to add an aqueous solution to the phospholipid /active principle complex. The volume of the aqueous solution has to be calculated according to the active principle sought concentration in the liposome solution.

The solvent is selected in function of the nature of the active principle(s) to be complexed with phospholipid . When feasible, it is advantageous to use solvents that are not to be removed afterward and may be present in the final product. If it is not possible the undesired solvent is removed from the complex medium, for instance, by evaporation or vacuum distillation.

As an example to illustrate the present invention, the procedures for the preparation of two complexes in gel, liquid or powder form are reported hereinafter. The relevant active principles are soluble in different solvents.

### EXAMPLE 1

### A - PHOSPHOLIPID /VITAMIN A COMPLEX IN GEL FORM

Vitamin A acetate in the exact calculated amount (10%, 1.000.000 Ul/g) was dissolved in hexane ( Vit. A / hexane 1:1). The mixture was stirred until a homogeneous mixture is obtained.

PHASE 2: Phospholipid powder (60 %), of the desired nature and purity, was added to the mixture of phase I, and a homogeneous mixture was obtained.

Hexane was removed from the complex under vacuum by increasing the temperature.

PHASE 3: When hexane is completely evaporated, ethanol (10%) is added thereto under stirring to obtain a homogeneous mixture.

PHASE 4: At the end glycerol is added to enhance the gel complex formation.

### B - PHOSPHOLIPID /VITAMIN A LIOUID FORM COMPLEX

PHASE 1 and 2: as indicated under A above (gel form)

PHASE 3: When hexane is completely evaporated ethanol is added in such an amount to obtain a liquid product.

### C - PHOSPHOLIPID /VITAMIN A POWDER FORM COMPLEX

PHASE 1,2 and 3: As indicated under A (Gel form)

PHASE 4: The obtained mixture was vacuum dried at room temperature.

### EXAMPLE 2

### A - PHOSPHOLIPID /SUPEROXIDE DISMUTASE (SOD) GEL FORM COMPLEX

PHASE 1: The desired amount of SOD (1.000.000 Ul/Kg) was dissolved in an alcoholic solution (water/ethanol ratio 1:3, amount 20%) under stirring until a homogeneous mixture was obtained.

PHASE 2: Phospholipid powder (70 %), of the desired nature and purity, was added to the mixture of phase 1, and a homogeneous mixture was obtained.

PHASE 3: In the preparation of this complex, as well as any complex whose active principles is water or water-ethanol soluble, phase 3 is not required. Ethanol, necessary for the double-layer liposome formation, is the one used to dissolve the active principle

PHASE 4: At the end glycerol is added to enhance the gel complex formation.

### B - PHOSPHOLIPID /SUPEROXIDE DISMUTASE (SOD) LIQUID FORM COMPLEX

PHASE 1 and 2: As indicated under A above (gel form)

PHASE 3: Ethanol is added thereto in such an amount to obtain a liquid product.

### C - PHOSPHOLIPID /SUPEROXIDE DISMUTASE (SOD) POWDER FORM COMPLEX

PHASE 1,2 and 3: As indicated under A (gel form)

PHASE 4: The obtained mixture was vacuum dried at room temperature.

### EXAMPLE 3

Measurement of entrapment efficiency.

A gelified phospholipid /Vitamin A complex was prepared according to the procedure described in Example 1A.

Liposomes were then obtained by adding a physiologic solution to the complex and subsequent stirring of the mixture.

The obtained liposomes have been subjected to ultracentrifugation in order to separate the liposome phase from the water phase. In both phases Vitamin A was determined as follows.

Liposome phase:

Phospholipid /vitamin A pellet resulting from the ultracentrifugation was dissolved in hexane, filtered under vacuum (0.2 .10⁵ Pa) and determined by HPLC (high performance liquid chromatography) using two different HPLC columns:
1) Column: Chromsep Nucleosil C18 100 mm x 3 ID
   Eluent: methanol
   Flow: 1 ml/min.
   Detecting wl: 325 nm
2) Column: Pertsil 10 ODS
   Eluent: Methanol/water (90/10)
   Flow: 1ml/min.
   Detecting wl: 325 nm

The water phase coming from the ultracentrifugation was analyzed for its vitamin A content using the same HPLC procedure and columns.

No Vitamin A was detected in the aqueous phase while almost all Vitamin A was detected in the liposome phase.

The above test confirms the high entrapment efficiency of the liposome obtained from the complexes manufactured according to the present invention.

## Claims

1. Process for the preparation of a phospholipid /active principle complex useful for the manufacture of one or more active substances containing liposome, wherein the active principle, intrinsically bound to the phospholipid molecules, is entrapped within liposomes comprising the following steps:
a) dissolving the active principle(s) into a solvent,
b) phospholipid addition to the above solution and solvent removing, if necessary,
c) final treatment of the complex to obtain the desired form,
characterized in that an alcoholic solution is added to the mixture coming out of step b) to enable the above complex molecules to reorganise in a multilamellar structure having a plurality of bimolecular layers.

2. Process according to claim 1, characterized in that the final treatment of the complex consists of a glycerol addition to enhance the gel formation.

3. Process according to claim 1, chatacterized in that the complex final treatment consists of an alcohol solution addition until a liquid product is obtained.

4. Process according to claim 1, characterized in that the complex final treatment consists in drying the same to obtain a complex in powder form.

5. Process according to anyone of the previous claims, characterized in that the solvent used for the complex multilamellar structure formation is the same solvent used to dissolve the active principle.

6. Phospholipid complex with one or more active principles prepared by the process according to claims 1 to 5, characterized in that the active principles are selected among the ones exhibiting activity in the cosmetic, pharmaceutical, dietetic and agronomic fields.

7. Phospholipid complex with one or more active principles prepared by the process according to claims 1 to 5, characterized in that the active principles are selected among vitamins, enzymes, antibiotics and hormones and their mixture.

8. Complex according to claim 7, characterized in that the active principle is Vitamin A.

9. Complex according to claim 7 characterized in that the active principle is Superoxide Dismutase.

10. Complex according to claim 7, characterized in that the phospholipids are of vegetal origin.

11. Compositions for pharmaceutical, cosmetic, dietetic and agronomic use containing the phospholipid /active principle complex according to claims 6 to 10.

## Patentansprüche

1. Verfahren zur Herstellung eines Phospholipid/Wirkstoff-Komplexes, der für die Herstellung eine oder mehrere Wirksubstanzen enthaltendem Liposom nützlich ist, wobei der Wirkstoff, welcher intrinsisch an den Phospholipidmolekülen gebunden ist, in Liposomen eingeschlossen ist, umfassend die folgenden Schritte:
a) Lösen des/der Wirkstoffe(s) in einem Lösungsmittel,
b) Phospholipidzugabe zu der obenstehenden Lösung und Lösungsmittelentfernung, sofern erforderlich,
c) Endbehandlung des Komplexes unter Erhalt der gewünschten Form,
dadurch gekennzeichnet, dass eine alkoholische Lösung der aus Schritt b) kommenden Mischung zugesetzt wird, um zu ermöglichen, dass sich die obenstehenden Komplexmoleküle zu einer multilamellaren Struktur mit einer Vielzahl an bimolekularen Schichten neu formieren.

2. Verfahren nach Anspruch 1, dadurch gekennzeichnet, dass die Endbehandlung des Komplexes aus einer Glycerolzugabe besteht, um die Gelbildung zu verstärken.

3. Verfahren nach Anspruch 1, dadurch gekennzeichnet, dass die Komplexendbehandlung aus einer Zugabe von Alkohollösung besteht, bis ein flüssiges Produkt erhalten wird.

4. Verfahren nach Anspruch 1, dadurch gekennzeichnet, dass die Komplexendbehandlung im Trocknen von selbigem besteht, um einen Komplex in Pulverform zu erhalten.

5. Verfahren nach mindestens einem der vorhergehenden Ansprüche, dadurch gekennzeichnet, dass das für die Bildung der multilamellaren Komplexstruktur verwendete Lösungsmittel das gleiche Lösungsmittel ist, das zur Lösung des Wirkstoffs verwendet wird.

6. Phospholipidkomplex mit einem oder mehreren Wirkstoffen, hergestellt durch das Verfahren gemäss den Ansprüchen 1 bis 5, dadurch gekennzeichnet, dass die Wirkstoffe unter jenen ausgewählt werden, die auf den Gebieten der Kosmetik, Pharmazie, Diätetik und Agronomie Wirksamkeit zeigen.

7. Phospholipidkomplex mit einem oder mehreren Wirkstoffen, hergestellt durch das Verfahren gemäss den Ansprüchen 1 bis 5, dadurch gekennzeichnet, dass die Wirkstoffe aus Vitaminen, Enzymen, Antibiotika und Hormonen und ihrer Mischung gewählt sind.

8. Komplex gemäss Anspruch 7, dadurch gekennzeichnet, dass der Wirkstoff Vitamin A ist.

9. Komplex gemäss Anspruch 7, dadurch gekennzeichnet, dass der Wirkstoff Superoxid-Dismutase ist.

10. Komplex gemäss Anspruch 7, dadurch gekennzeichnet, dass die Phospholipide pflanzlichen Ursprungs sind.

11. Zusammensetzungen für die pharmazeutische, kosmetische, diätetische und agronomische Anwendung, enthaltend den Phospholipid/Wirkstoff-Komplex gemäss den Ansprüchen 6 bis 10.

## Revendications

1. Procédé de préparation d'un complexe de principe actif phospholipide utile pour la fabrication d'un liposome contenant une ou plusieurs substance(s) active(s), dans lequel le principe actif, lié de manière intrinsèque aux molécules de phospholipide, est encapsulé dans des liposomes et comprenant les étapes suivantes :
a) dissolution du ou des principe(s) actif(s) dans un solvant,
b) addition du phospholipide à la solution précédente et élimination du solvant, si nécessaire,
c) traitement final du complexe pour obtenir la forme souhaitée,
caractérisé en ce qu'une solution alcoolique est ajoutée au mélange résultant de l'étape b) pour permettre aux molécules du complexe ci-dessus de se réarranger en une structure multilamellaire présentant une pluralité de couches bimoléculaires.

2. Procédé selon la revendication 1, caractérisé en ce que le traitement final du complexe consiste en une addition de glycérine pour améliorer la formation de gel.

3. Procédé selon la revendication 1, caractérisé en ce que le traitement final du complexe consiste en une addition de solution d'alcool jusqu'à l'obtention d'un produit liquide.

4. Procédé selon la revendication 1, caractérisé en ce que le traitement final du complexe consiste en un séchage de celui-ci pour obtenir un complexe sous forme de poudre.

5. Procédé selon l'une quelconque des revendications précédentes, caractérisé en ce que le solvant utilisé pour la formation de structure multilamellaire du complexe est le même solvant que celui utilisé pour dissoudre le principe actif.

6. Complexe de phospholipide avec un ou plusieurs principe(s) actif(s) préparé en utilisant le procédé selon les revendications 1 à 5, caractérisé en ce que les principes actifs sont choisis parmi ceux présentant une activité dans les domaines cosmétique, pharmaceutique, diététique et agronomique.

7. Complexe de phospholipide avec un ou plusieurs principe(s) actif(s) préparé en utilisant le procédé selon les revendications 1 à 5, caractérisé en ce que les principes actifs sont choisis parmi les vitamines, les enzymes, les antibiotiques et les hormones, ainsi que parmi leur mélange.

8. Complexe de phospholipide selon la revendication 7, caractérisé en ce que le principe actif est de la vitamine A.

9. Complexe de phospholipide selon la revendication 7, caractérisé en ce que le principe actif est de la superoxyde-dismutase.

10. Complexe de phospholipide selon la revendication 7, caractérisé en ce que les phospholipides sont d'origine végétale.

11. Compositions pour usage pharmaceutique, cosmétique, diététique et agronomique contenant le complexe de principe actif phospholipide selon les revendications 6 à 10.
